# EUROPÄISCHE PATENTSCHRIFT

(11) **EP 0 844 303 B1**
(45) Veröffentlichungstag und Bekanntmachung des Hinweises auf die Patenterteilung: **04.05.2005**
(21) Anmeldenummer: 97119119.2
(22) Anmeldetag: 03.11.1997
(51) Int. Cl.: C11D 17/00, C11D 3/382, C11D 3/20, C11D 1/90, A61L 9/01

(54) **Stückförmiges Mittel für den Toilettenbereich**
Solid article for use in toilets
Article solide pour les WC

(30) Priorität: 25.11.1996 DE 19648788
(43) Veröffentlichungstag der Anmeldung: 27.05.1998
(73) Patentinhaber: Buck-Chemie GmbH ., 71083 Herrenberg (DE)
(72) Erfinder: Dettinger, Johannes Dr. Dipl.-chem., 72108 Rottenburg (DE); Jaeschke, Edgar Dipl.-Ing, 70794 Filderstadt (DE)
(74) Vertreter: Mammel, Ulrike

(56) Entgegenhaltungen:
- EP-A- 0 268 967
- GB-A- 2 061 313
- US-A- 5 562 850
- DATABASE WPI Section Ch, Week 8431 Derwent Publications Ltd., London, GB; Class A97, AN 84-191818 XP002087327 & JP 59 108100 A (FUJISAWA PHARM CO LTD) , 22. Juni 1984
- GERLING K -G: "LACTOBIONSAURE UND DEREN DERIVATE ALS WASCHMITTELROHSTOFFE LACTOBIONSAURE, COBUILDER, LACTOBIONAMIDE, TENSIDE" SOFW-JOURNAL SEIFEN, OELE, FETTE, WACHSE, Bd. 121, Nr. 11, 1. Oktober 1995, Seiten 804- 812, XP000529105
- DATABASE WPI Section Ch, Week 8725 Derwent Publications Ltd., London, GB; Class D22, AN 87-172825 XP002087328 & JP 62 102760 A (AIKOH CO LTD) , 13. Mai 1987
- LOMAX E.R.: 'AMPHOTERIC SURFACTANTS', 1996, MARCEL DEKKER, INC, N. YORK, BASEL, HONG KONG

## Beschreibung

Die Erfindung betrifft ein stückförmiges Mittel für den Toilettenbereich sowie ein Verfahren zur Herstellung des stückförmigen Mittels. Grundsätzlich kann das stückförmige Mittel ein Reinigungsmittel in Form eines Reinigungsblocks oder ein Duftmittel in Form eines Duftspenders sein.

Mittel der genannten Art, die Tenside umfassen, sind im Stand der Technik in unterschiedlichen Zusammensetzungen bekannt. Durch das steigende Umweltbewußtsein der Verbraucher sind die Zusammensetzungen dieser Mittel immer mehr dahingehend modifiziert worden, daß umweltschädliche Bestandteile durch umweltverträglichere ersetzt oder, soweit möglich, ganz aus der Zusammensetzung weggelassen wurden. Daher bestand in den letzten Jahren eine Tendenz, beispielsweise Schaumbildner aufgrund umweltbedingter Risiken nicht mehr in Reinigungsblökken oder Duftspendern für den Toilettenbereich zu verwenden. Das Weglassen solcher Schaumbildner hat sich als nachteilig erwiesen. Zum einen wird den Verbrauchern damit eine schnelle Überprüfungsmöglichkeit genommen, ob das im Toilettenbereich eingesetzte Mittel bereits aufgebraucht ist. Zum anderen hat sich gezeigt, daß die Verbraucher die Schaumbildung als Maß für die Reinigungskraft des eingesetzten Mittels ansehen und daher von solchen Reinigungsblöcken oder Duftspendern, die keinen oder nur sehr geringen Schaum entwickeln, hinsichtlich deren Wirksamkeit nicht überzeugt sind.

Daher liegt der vorliegenden Erfindung die Aufgabe zugrunde, ein Mittel für den Toilettenbereich bereitzustellen, das einen Bestandteil aufweist, welcher eine gleichmäßige Schaumentwicklung gewährleistet, dabei im wesentlichen umweltverträglich ist und verglichen mit herkömmlichen Schaumbildnern in geringerer Menge zugesetzt werden muß.

Diese Aufgabe wird gelöst durch die Merkmale des Anspruchs 1. Weitere bevorzugte Ausführungsformen sind den Ansprüchen 2-13 zu entnehmen

Dabei kann als Schaumbildner ein Betain gemäß Anspruch 1 eingesetzt werden. Betaine weisen ja bekanntlich eine Carboxygruppe und einen quartären Stickstoff auf und sind zwitterionische Verbindungen folgender allgemeiner Strukturformel: 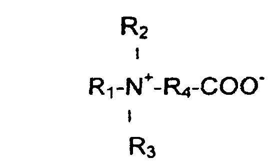 wobei jeder der Reste R₁, R₂, R₃ und R₄ Kohlenstoff und Wasserstoff umfaßt. Die erfindungsgemäßen Betaine weisen an wenigstens einem der Reste R₁, R₂, R₃ oder R₄ zusätzlich ein Heteroatom auf. Unter Heteroatom werden beispielsweise Sauerstoff, Stickstoff, Phosphor, Schwefel oder die Halogene verstanden. Bevorzugt ist, daß einer der Reste R₁, R₂ oder R₃ zwei Heteroatome, vorzugsweise Stickstoff und Sauerstoff in Form einer Amidgruppe aufweist, die über eine Alkylgruppe mit dem quartären Stickstoff verbrückt ist.

Betaine sind in verdünnter Lösung leicht biologisch abbaubar. Daneben besitzen sie besonders günstige oberflächenaktive Eigenschaften, sind in der Regel starke Schäumer und zugleich hervorragende Reinigungsmittel. In saurer Lösung kann man sogar die dann auftretende bakterizide Wirkung ausnutzen.

Bei der Verwendung zumindest eines Lactobionsäurederivates ergibt sich der Vorteil, daß ein Wirkstoff zur Verfügung steht, der sogar als nachwachsender Rohstoff erhältlich ist. Außerdem kann dieser Zusatz schon bei Einsatz sehr geringer Mengen eine deutliche Verbesserung des Schaumvermögens erzielen. Gegenüber den üblicherweise eingesetzten gewichtsprozentualen Anteilen an Schaumbildnern läßt sich die Menge an zugesetztem Lactobionsäurederivat bei gleicher Schaumwirkung im Vergleich zum Stand der Technik um in etwa die Hälfte reduzieren.

Neben dem Schaumvermögen zeigt dieser Zusatz auch ein deutlich verbessertes Netzvermögen. Der gebildete Schaum ist feinblasig und zeitstabil. Es hat sich auch als vorteilhaft erwiesen, in der Formulierung des Mittels das zumindest eine Betain mit einem Lactobionsäurederivat zu kombinieren. Dabei ergänzen sich die vorteilhaften Eigenschaften des eingesetzten Betains mit denen des Lactobionsäurederivats. Darüber hinaus ist es auch möglich, Lactobionsäure zuzusetzen.

Das erfindungsgemäße stückförmige Mittel kann ein Reinigungsmittel in Form eines Reinigungsblocks oder ein Duftmittel in Form eines Duftspenders sein. Beide Mittel für den Toilettenbereich haben im wesentlichen die gleiche Zusammensetzung. Das Duftmittel unterscheidet sich von dem Reinigungsmittel im wesentlichen durch einen höheren Anteil an Parfum und weist in der Regel auch einen Farbbestandteil auf.

Das Betain, das ein amphoteres Tensid ist, kann ein Fettsäureamidopropyl-Betain sein, dessen Fettsäure einen Alkyl- oder Alkenylrest mit 5 bis 21 Kohlenstoffatomen aufweist. Für die Erfordernisse des erfindungsgemäßen Mittels hat es sich als günstig erwiesen, als Fettsäure des Betains Kokosfettsäure zu wählen. Dabei entspricht die Benennung des Betains als Kokosfettsäure-amidopropyl-Betain in etwa der CTFA-Nomenklatur. Unter CTFA versteht man die Cosmetics, Toiletry and Fragrance Association. Die genaue Bezeichnung des bevorzugten Betains in der CTFA-Nomenklatur lautet Cocamidopropyl Betaine, die chemische Bezeichnung Dimethylcarboxymethyl-kokosfettsäure-amidoammonium-betain.

Wenn für das Mittel ein Zusatz in Form von zumindest einem Lactobionsäurederivat gewählt wird, so wird das Lactobionsäurederivat vorzugsweise aus der Gruppe der Lactobionofettsäureamide ausgewählt und ist besonders bevorzugt Lactobionococylamid, Lactobionooleylamid oder Lactobionotalgamid oder eine Mischung dieser Lactobionofettsäureamide. Es können aber genauso andere Lactobionofettsäureamide oder -derivate erfindungsgemäß eingesetzt werden.

Das erfindungsgemäße Mittel enthält 10 bis 60 Gew%, vorzugsweise 10 bis 50 Gew% anionisches Tensid (a). Dabei finden insbesondere solche, ausgewählt aus der Gruppe bestehend aus Alkylsulfat, Fettalkoholsulfat, Fettalkoholethersulfat und Alkylbenzolsulfonat oder deren Mischungen Verwendung. Dabei sind Alkylkettenlängen der Alkylreste oder Fettsäurebestandteile von C₈-C₁₈ bevorzugt.

Nichtionische Tenside (b) können wahlweise eingesetzt werden. Hier eignen sich besonders die Anlagerungsprodukte von 3 bis 80 Mol Ethylenoxid an 1 Mol einer aliphatischen Verbindung mit im wesentlichen 8 bis 20 Kohlenstoffatomen, die ausgewählt sind aus der Gruppe der Alkohole und Fettsäuren. Aber auch alkylaromatische Verbindungen, wie Alkylphenole, sind hier geeignet. Der Anteil der nichtionischen Tenside in dem erfindungsgemäßen Mittel kann bis zu 20 Gew% betragen.

Bei den amphoteren Tensiden (c) werden 0 bis 10 Gew% eingesetzt, vorzugsweise sind es 0 bis 5 Gew%, insbesondere zwischen 0,1 Gew% und 4 Gew%, bevorzugt zwischen 0,3 Gew% und 3 Gew% und besonders bevorzugt zwischen 0,5 Gew% und 2,5 Gew% Betain, das zusätzlich zu der Carboxygruppe und dem quartären Stickstoffatom an wenigstens einem Rest wenigstens ein Heteroatom aufweist. Die obigen Gewichtsprozentangaben beziehen sich auf die Masse der Betaine als Reinsubstanz.

Von diesen Betainen sind die eine alkylverbrückte Amidfunktion umfassenden Betaine, insbesondere die Fettsäure-amidopropyl-Betaine mit einem C₅-C₂₁-Fettsäureanteil besonders bevorzugt. Generell ist es vorteilhaft, daß die amphoteren Tenside beliebig mit dem jeweils verwendeten anionischen und gegebenenfalls nichtionischen Tensid oder den anionischen und gegebenenfalls nichtionischen Tensiden kombinierbar sind.

Ein weiterer Vorteil der erfindungsgemäßen Betaine besteht darin, daß sie als konzentrierte wäßrige Lösungen mit einem Wassergehalt von weniger als 60 Gew%, vorzugsweise mit weniger als 55 Gew% Wasser, bereits so dünnflüssig sind, daß sie mit den anderen Bestandteilen der Zusammensetzung gemischt und extrudiert werden können. Hierdurch wird der Wasseranteil des Mittels nicht unnötig erhöht, und es kann ein kompaktes Mittel in hoher Wirkkonzentration bereitgestellt werden.

Der Reinigungsblock kann 0 bis 5 Gew% zumindest eines Lactobionsäurederivats (d) enthalten. Bei einem zumindest ein Lactobionsäurederivat enthaltenden Mittel sind Konzentrationsbereiche zwischen 1 und 4 Gew. %, insbesondere zwischen 1 und 2,5 Gew%, bevorzugt.

Der Gesamtanteil der Komponenten (c) und (d) sollte 0,5 bis 10 Gew% betragen. Die Komponenten (c) und/oder (d) dienen im wesentlichen als Schaumbildner. Bei Verwendung von Lactobionsäurederivaten als Schaumbildner kann man bei gleicher Schaumwirkung die zugesetzte Menge an Schaumbildnern gegenüber herkömmlichen Formulierungen um etwa die Hälfte oder mehr reduzieren.

Das Mittel enthalt 20-80 Gew. % anorganische Salze Das Mittel gemäß dieser bevorzugten Ausführungsform kann 20 bis 80 Gew% anorganisches Salz (e), vorzugsweise 40 bis 75 Gew% eines anorganischen Salzes, das ausgewählt ist aus der Gruppe bestehend aus den Alkali- und/oder Erdalkalisalzen der Schwefelsäure, Phosphorsäuren, Stickstoffsäuren, Kohlensäure oder Salzsäure oder deren Mischungen, enthalten. Dadurch läßt sich die Konsistenz und das Abspülverhalten des Reinigungsblockes günstig beeinflussen.

In dem Mittel ist auch Parfum (f) in einem Anteil von bis zu 10 Gew%, vorzugsweise 2 bis 7 Gew%, enthalten. Dadurch wird die Desodorierung des Toilettenbereiches bewirkt. Gleichzeitig kann das Parfum dann, wenn es in Form von flüssigen Parfumölen eingesetzt wird, aber auch die Konsistenz des fertigen Reinigungsblokkes mitbestimmen und trägt insbesondere zur Plastifizierung der Rohmasse bei.

Als weiteren wahlweisen Bestandteil kann das erfindungsgemäße Mittel Farbe (g) enthalten. Als Farbbestandteil eignen sich grundsätzlich Pigmente und Farbstoffe. Wenn man erreichen will, daß das erfindungsgemäße Mittel, wenn es z. B. als Reinigungsblock eingesetzt wird, als solches farbig sein soll, wird bevorzugt Farbe in Form von Pigmenten zugesetzt. Andererseits kann es aber auch erwünscht sein, daß die von dem Reinigungsblock während eines Spülvorgangs abgelösten Bestandteile in Form einer farbigen Lösung für den Verbraucher sichtbar werden. Dies ist z. B. bevorzugt der Fall, wenn das erfindungsgemäße Mittel im wesentlichen als Duftspender eingesetzt wird, wobei der Duftspender dann aufgrund der enthaltenen oberflächenaktiven Stoffe in Form der Tenside selbstverständlich auch eine Reinigungswirkung entfaltet. Um eine solche farbige Lösung zu erzielen, wird man bevorzugt Farbstoffe als Bestandteil des Mittels wählen. Auch eine Kombination von Pigment(en) und Farbstoff(en) ist möglich.

Dem Mittel können auch 0 bis 30 Gew% Desinfektionsmittel (h) beigefügt werden. Vorzugsweise werden hierfür Chlorisocyanurate, wie beispielsweise Natriumdichlorisocyanurat oder andere chlorabspaltende Reagenzien sowie Phenole verwendet. Als chlorabspaltendes Reagenz kann beispielsweise Triclosan Verwendung finden, unter den Phenolen ist o-Phenylphenol zu nennen. Bei Einsatz von Triclosan oder eines Phenols können sehr viel geringere Mengen des Desinfektionsmittels verwendet werden als dies im Fall der Chlorisocyanurate erforderlich ist.

Als Bleichmittel (i) können 0 bis 25 Gew% eines Bleichmittels auf Sauerstoffbasis verwendet werden, wie z. B. die Natriumsalze der Percarbonate oder Perborate.

Als Aktivator (j) kann z. B. Tetraacetylethylendiamin (TAED) eingesetzt werden.

Das erfindungsgemäße Mittel kann 0 bis 5 Gew% Komplexierungsmittel (k) enthalten, die gegen die Wasserhärte und zur Komplexbildung mit Schwermetallionen eingesetzt werden. Die jeweiligen Mengen des Komplexierungsmittels werden entsprechend des Komplexierungsvermögens des Mittels und entsprechend der gewünschten Abspülgeschwindigkeit gewählt.

Um zu gewährleisten, daß das erfindungsgemäße Mittel eine definierte Standzeit aufweist, und daß pro Spülvorgang jeweils die gleichen Mengen von dem stückförmigen Mittel abgespült werden, können Abspülregulatoren (l) eingesetzt werden. Hierfür eignen sich insbesondere die Salze aromatischer Sulfonsäuren, beispielsweise Natriumcumolsulfonat oder Natriumtoluolsulfonat. Zu dem gleichen Zweck kann auch Dipropylenglykol eingesetzt werden. Wird die zuletzt genannte Verbindung eingesetzt, kann ihr Anteil an dem Reinigungsblock sogar äußerst gering gehalten werden. In aller Regel ist dann ein Anteil von bis zu 5 Gew% ausreichend. Im Bereich der Salze der aromatischen Sulfonsäuren beträgt der Anteil bis zu 20 Gew%, bezogen auf das fertige Mittel.

Kalk und Urinstein lösende Säuren (m) können dem Mittel zugesetzt werden, um zu verhindern, daß sich Kalksteinablagerungen in Toilettenschüsseln bilden oder um bereits vorhandene Ablagerungen wieder zu lösen. Hierfür eignen sich Säuren, wie Amidosulfonsäure und Zitronensäure sowie andere organische Säuren. Sie können bis zu einem Anteil von 40 Gew% in dem fertigen Mittel enthalten sein.

Des weiteren kann das erfindungsgemäße Mittel Extrusionshilfsmittel (n) enthalten. Diese dienen dazu, die Rohmasse des Mittels in der entsprechenden Zusammensetzung formen zu können. Geeignete Extrusionshilfsmittel sind beispielsweise Polyethylenglykol. In der Regel ist es ausreichend, bis zu 20 Gew% Extrusionshilfsmittel einzusetzen. Da aber auch weitere Bestandteile des Reinigungsblockes eine bessere Formbarkeit der Reinigungsmasse ermöglichen, kann der Anteil an zugesetztem Extrusionshilfsmittel sehr viel geringer als 20 Gew% sein oder es kann ganz auf den Zusatz eines solchen Mittels verzichtet werden. Zu den weiteren Bestandteilen der Reinigungsblockmasse, die auch die Formbarkeit erleichtern, gehört u. a. das als Abspülregulator fakultativ verwendbare Dipropylenglykol. Auch fakultativ einsetzbare Parfümöle oder flüssige Säuren dienen diesem Zweck.

Das erfindungsgemäße Mittel kann neben den bisher genannten Bestandteilen weitere an sich übliche Zusätze (o) bis zu einem Anteil von 5 Gew% enthalten.

Zu solchen weiteren Zusätzen gehören beispielsweise Enzyme, wie Savinase, Cellulase, Protease und Lipase sowie Konservierungsmittel.

Die Erfindung betrifft auch ein Verfahren zur Herstellung eines erfindungsgemäßen Mittels, bei dem eine Masse entsprechender geeigneter Zusammensetzung mittels des Strangpreßverfahrens hergestellt wird. Dabei wird die vorgemischte Masse mittels eines Extruders zu einem endlosen Strang geformt und dieser dann in Stücke oder Blöcke geeigneter Größe geschnitten. Die Größe der Stücke ist variabel und kann entsprechend des Einsatzzweckes gewählt werden. In der Regel werden die Stücke so geschnitten, daß sie nach dem Schneiden Blöcke mit einem Gewicht von in etwa 20 bis 100 g, vorzugsweise 20 bis 50 g ergeben.

### Beispiele

Stückförmige Mittel für den Toilettenbereich, die sowohl als Reinigungsblöcke wie auch als Duftspender einsetzbar sind, wurden entsprechend der nachfolgend in den Rezepturen angegebenen Zusammensetzungen durch Mischen der Bestandteile und anschließendes Extrudieren hergestellt. Die auf diese Weise durch Extrudieren hergestellten Endlosstränge wurden in einzelne Blöcke mit einem Gewicht von in etwa 50 g geschnitten.

Aufgrund des gewählten Zusatzes an Parfum und Farbe in Form eines Farbstoffes ist insbesondere Rezeptur II.3 für den Einsatz als Duftspender geeignet.

### I. Rezepturen auf Alkylbenzolsulfonat-Basis

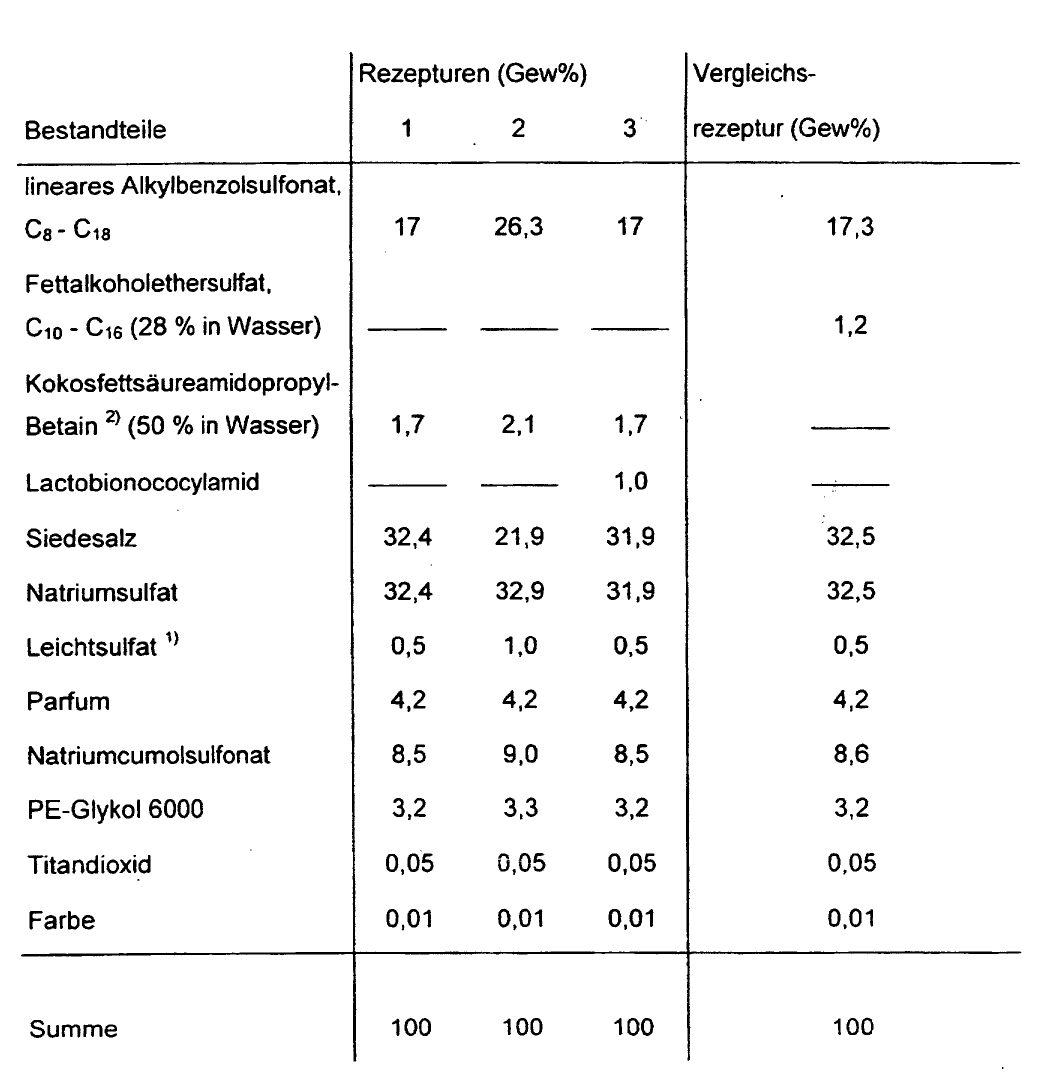

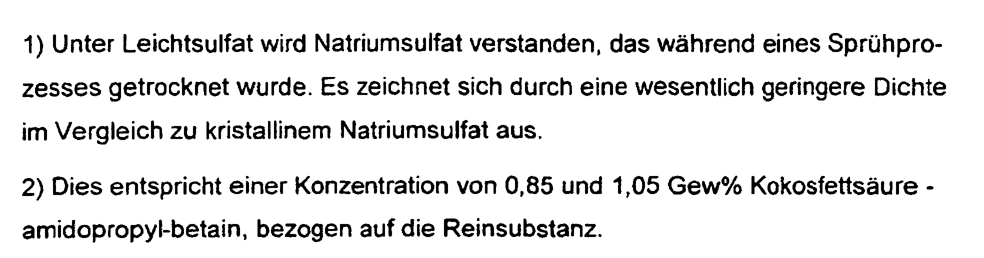

Für die gemäß den obengenannten Rezepturen hergestellten Reinigungsblöcke wurden die Schaumzahl und die Netzzahl nach dem weiter unten angegebenen Verfahren bestimmt. Die ermittelten Werte gibt Tabelle 1 wieder:

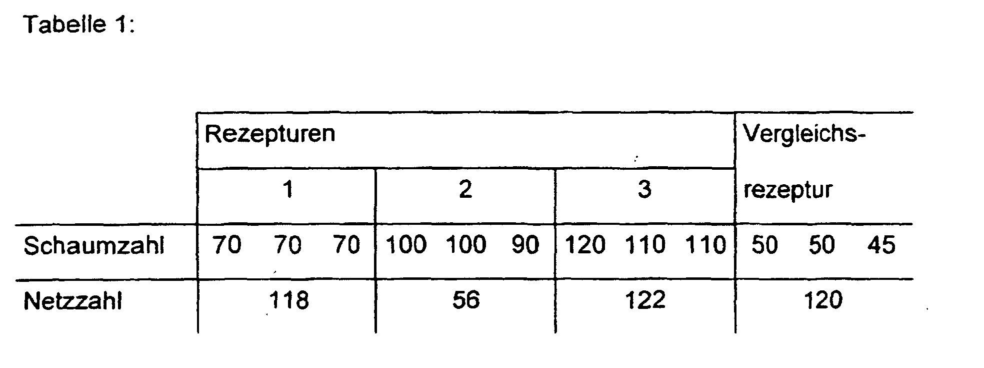

Durch die Verwendung insbesondere des Lactobionsäurederivates ergibt sich schon bei geringem Einsatz eine deutliche Verbesserung des Schaumvermögens in Form eines feinblasigen Schaums, der Zeitstabilität aufweist, wobei eine synergistische Wirkung der beiden zusammen in der dritten Rezeptor von I.1. verwendeten Schaumbildner - Kokosfettsäure-amidopropyl-Betain und Lactobionococylamid - festgestellt werden konnte.

Bezogen auf eine vergleichbare Schaumwirkung zu der Vergleichsrezeptur kann man den für das Schäumen verantwortlichen Bestandteil der dritten Rezeptur von I.1. um in etwa die Hälfte reduzieren und somit die Umweltverträglichkeit des Reinigungsblockes verbessern. Diese wird auch zusätzlich dadurch verbessert, daß der Wirkstoff aus nachwachsendem Rohstoff erhältlich ist.

### II. 1. Rezeptur auf Fettalkoholsulfat-Basis

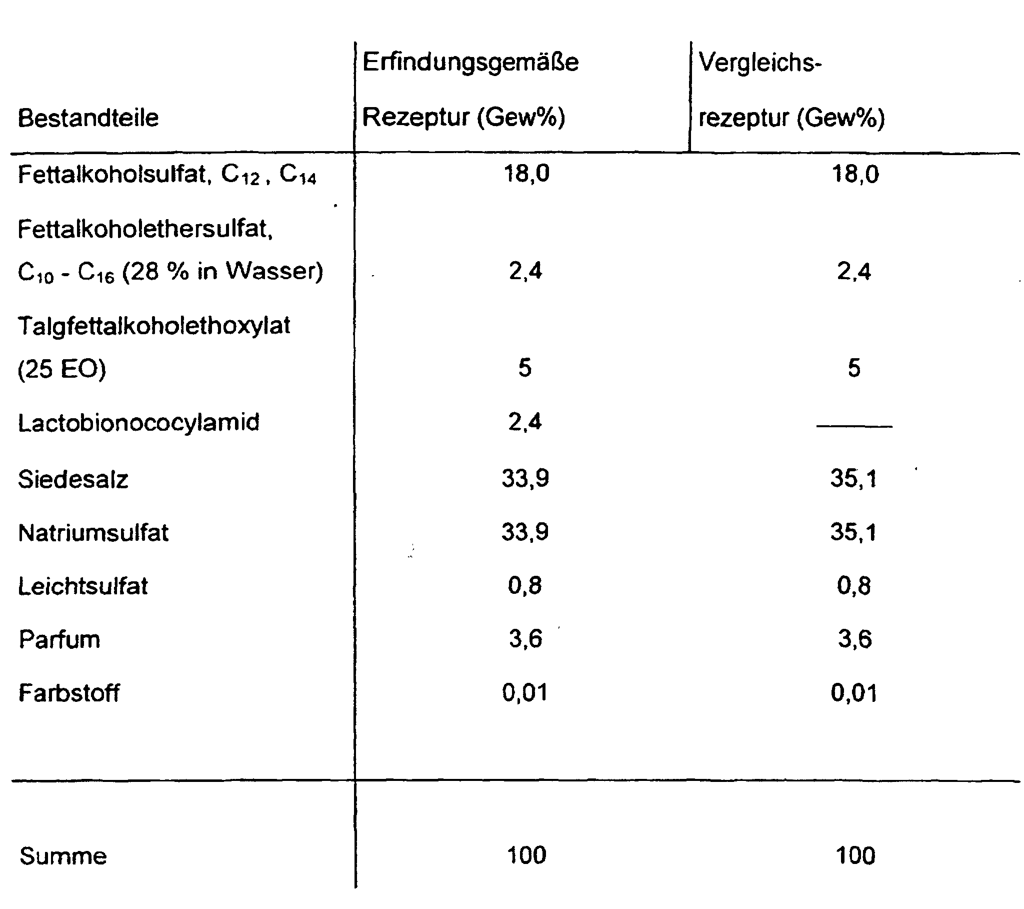

Die für diese Rezepturen ermittelten Schaumzahlen und Netzzahlen sind in Tabelle 2 angegeben.

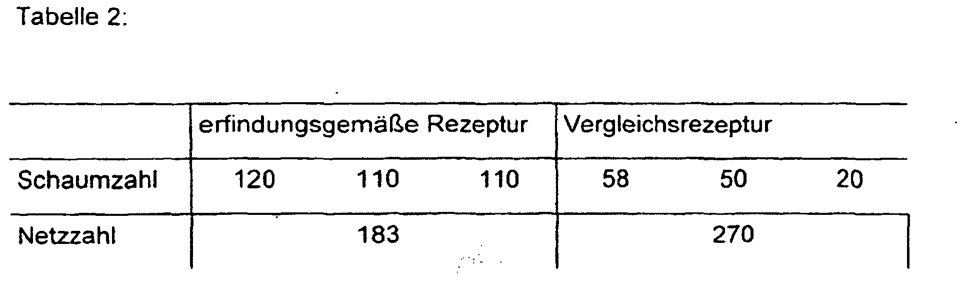

### II. 2. Rezeptur auf Fettalkoholsulfat-Basis

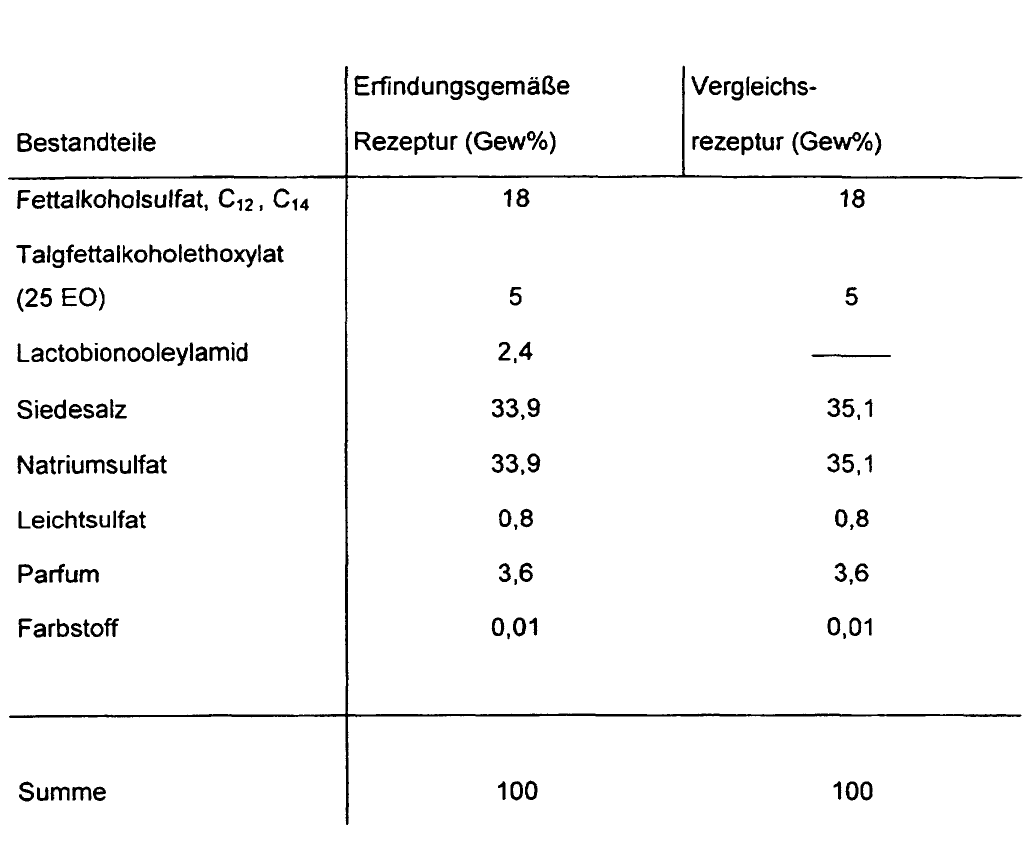

Die für diese Rezepturen ermittelten Schaumzahlen und Netzzahlen sind in Tabelle 3 angegeben.

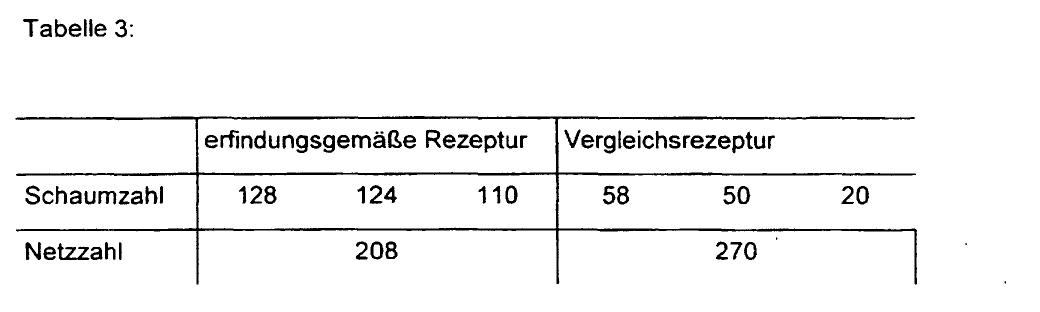

### II. 3. Rezeptur auf Fettalkoholsulfat-Basis

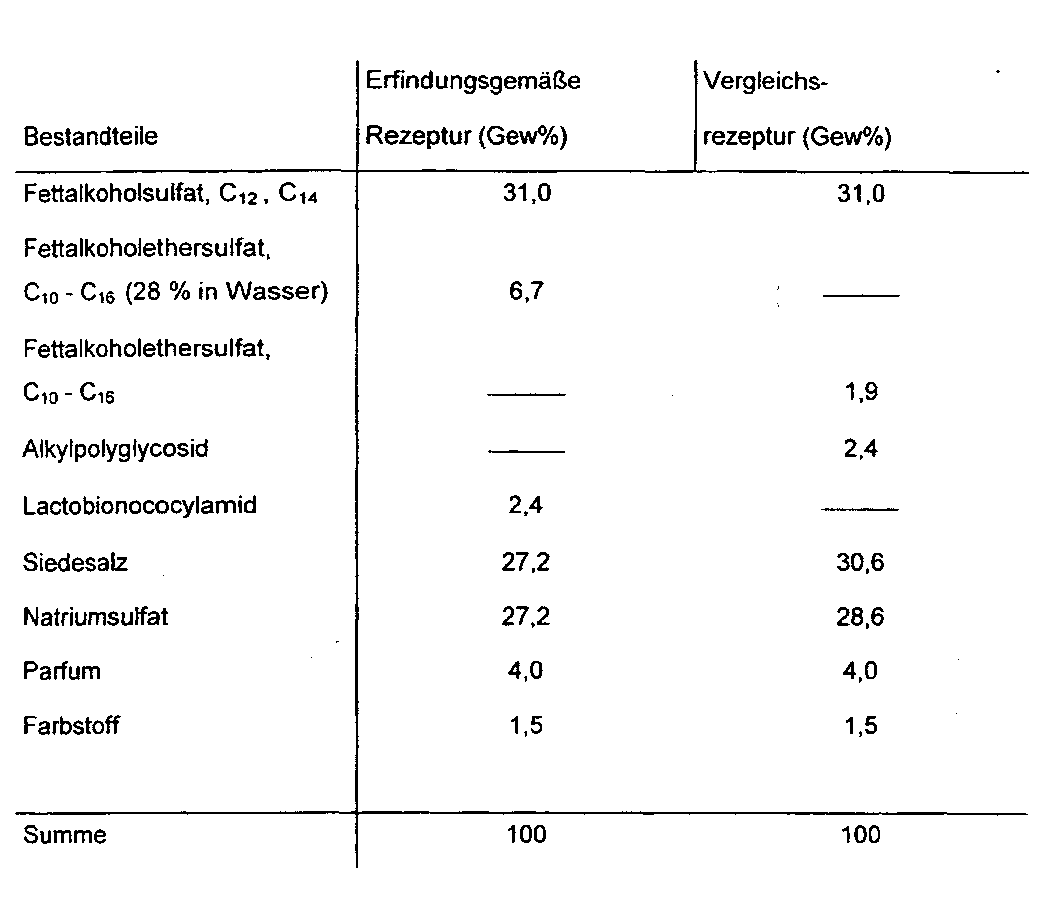

Die für diese Rezepturen ermittelten Schaumzahlen und Netzzahlen sind in Tabelle 4 angegeben.

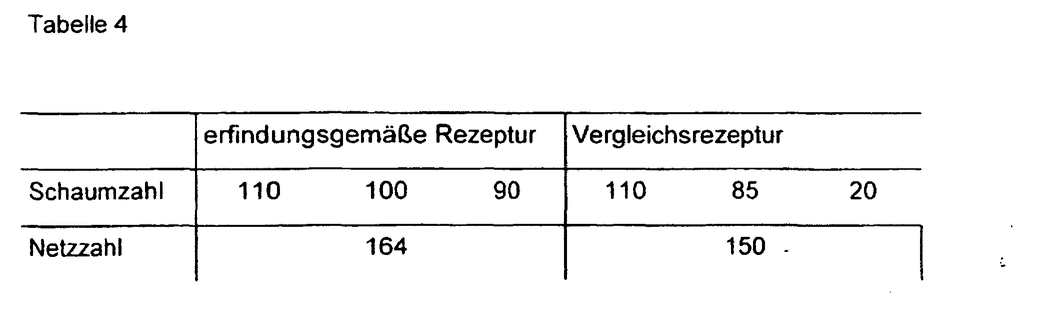

Die Tabellen 2 bis 4 zeigen, daß durch die Verwendung eines geringen Zusatzes des Lactobionsäurederivats eine deutliche Verbesserung des Schaumvermögens erreicht werden konnte. Es wurde bei den erfindungsgemäßen Rezepturen ein feinblasiger Schaum, der Zeitstabilität aufweist, erhalten. Da nur eine geringe Menge an Lactobionococylamid oder Lactobionooleylamid zur Erzielung des guten Schaumvermögens erforderlich ist, wird die Umweltverträglichkeit des so hergestellten Reinigungsblockes verbessert. Hinzu kommt noch als weiterer positiver Aspekt, daß der Wirkstoff aus nachwachsenden Rohstoffen erhältlich ist.

Insbesondere Rezeptur II.3 ist aufgrund ihres relativ hohen Parfum- und Farbstoffgehaltes zur Verwendung als Duftmittel im Toilettenbereich gut geeignet.

### III. Ermittlung der Schaumzahl und der Netzzahl.

Hierfür werden in einen 1 l Meßkolben 1,00 g des zu überprüfenden Toilettenreinigungsmittels eingewogen, mit Leitungswasser (20 °C) auf 1 l aufgefüllt und unter Rühren gelöst. Die so hergestellte Lösung wird im folgenden als Stammlösung bezeichnet.

### III.1 Ermittlung der Schaumzahl über das Schaumvermögen.

100 ml Stammlösung werden in einen 250 ml Mischzylinder überführt und mit einem PTFE-Stopfen verschlossen. Dann wird der Zylinder zwanzigmal geschüttelt. Nach 30 Sekunden, 5 Minuten und 30 Minuten wird das erzeugte Schaumvolumen (ml) abgelesen.

### III.2. Ermittlung der Netzzahl über das Netzvermögen.

500 ml der Stammlösung werden in ein 800 ml Becherglas überführt. Dann wird in Anlehnung an DIN 53901 ein Baumwollplättchen in die Lösung eingebracht und die Zeit bis zum Absinken des Netzplättchens gemessen. Bei Meßwerten ohne nennenswerte Schwankungen wird diese Zeitmessung fünfmal durchgeführt, bei schwankenden Meßwerten zehnmal. Der niedrigste und der höchste Wert werden verworfen und aus den restlichen Meßwerten wird die Netzzahl als Mittelwert gebildet.

Hinsichtlich des Plättchensinkvermögens gilt, daß die niedrigere Netzzahl grundsätzlich die bessere ist.

## Patentansprüche

1. Stückförmiges Mittel für den Toiletten bereich , welches Mittel 10 bis 60 Gew.% anionische Tenside, 20 - 80 Gew. % anorganische Salze, Parfüm und wenigstens ein Lactobionofettsäureamid und/oder ein Betain enthält, wobei das Betain zusätzlich zu der Carboxygruppe und dem quartären Stickstoffatom an wenigstens einem Rest wenigstens ein Heteroatom aufweist und das Mittel extrudiert ist.

2. Mittel nach Anspruch 1, **dadurch gekennzeichnet, dass** es ein Reinigungsmittel in Form eines Reinigungsblockes ist.

3. Mittel nach Anspruch 1, **dadurch gekennzeichnet, dass** es ein Duftmittel in Form eines Duftspenders ist.

4. Mittel nach einem der Ansprüche 1 bis 3, **dadurch gekennzeichnet, dass** das Betain eine alkylverbrückte Amidfunktion umfasst.

5. Mittel nach einem der Ansprüche 1 bis 4, **dadurch gekennzeichnet, dass** das Betain ein Fettsäure-amidopropyl-Betain ist, wobei die Fettsäure einen Alkyl- oder Alkenylrest mit 5 - 21 Kohlenstoffatomen aufweist.

6. Mittel nach Anspruch 5, **dadurch gekennzeichnet, dass** die Fettsäure Kokosfettsäure ist.

7. Mittel nach Anspruch 1, **dadurch gekennzeichnet, dass** das Lactobionofettsäureamid Lactobionococylamid, Lactobionooleylamid, Lactobionotalgamid oder eine Mischung davon ist.

8. Mittel nach einem der Ansprüche 1 bis 7 bestehend aus
a) 10 - 60 Gew% anionischem Tensid,
b) 0 - 20 Gew% nichtionischem Tensid,
c) 0 - 10 Gew% amphoterem Tensid,
d) 0 - 5 Gew% zumindest eines Lactobionofettsäureamids,
e) 20 - 80 Gew% anorganischem Salz
f) bis zu 10 Gew% Parfum,
g) 0 - 5 Gew% Farbe,
h) 0 - 30 Gew% Desinfektionsmittel,
i) 0 - 25 Gew% Bleichmittel
j) 0 - 7 Gew% Aktivator,
k) 0 - 5 Gew% Komplexierungsmittel
l) 0 - 20 Gew% Abspülregulator,
m) 0 - 40 Gew% Kalk- und Urinstein lösender Säure,
n) 0 - 20 Gew% Extrusionshilfsmittel,
o) 0 - 5 Gew% weiteren Zusätzen,
wobei der Gesamtanteil der Komponenten c) und d) 0,5 bis 10 Gew% beträgt.

9. Mittel nach einem der Ansprüche 1 bis 8, bestehend aus
a) 10 - 50 Gew% anionischem Tensid, ausgewählt aus der Gruppe bestehend aus Alkytsulfat, Fettalkoholsulfat, Fettalkoholethersutfat und Alkylbenzolsulfonat oder deren Mischungen,
b) 0 - 20 Gew% nichtionischem Tensid, ausgewählt aus der Gruppe der Anlagerungsprodukte von 3 - 80 Mol Ethylenoxid an langkettige aliphatische Alkohole und Fettsäurealkohole mit 8 - 20 C-Atomen,
c) 0 - 5 Gew% Fettsäure-amidopropyl-Betain mit einem C₅-C₂₁-Fettsäureanteil,
d) 0 - 5 Gew% zumindest eines Lactobionofettsäureamids
e) 40 - 75 Gew% anorganischem Salz, ausgewählt aus der Gruppe bestehend aus den Alkali- und/oder Erdalkalisalzen der Schwefelsäure, Phosphorsäuren, Stickstoffsäuren, Kohlensäure oder Salzsäure oder deren Mischungen,
f) 2 - 7 Gew% Parfum
g) 0 - 5 Gew% Farbe,
h) 0 - 30 Gew% Desinfektionsmittel
i) 0 - 25 Gew% Bleichmittel,
j) 0 - 7 Gew% Aktivator,
k) 0 - 5 Gew% Komplexierungsmittel
l) 0 - 20 Gew% Abspütregutator,
m) 0 - 40 Gew% Kalk- und Urinstein lösender Säure,
n) 0 - 20 Gew% Extrusionshilfsmittel
o) 0 - 5 Gew% weiteren Zusätzen,
wobei der Gesamtanteil der Komponenten c) und d) 0,5 - 10 Gew % beträgt.

10. Mittel nach einem der Ansprüche 3 bis 9 bestehend aus
a) 10 - 50 Gew% anionischem Tensid, ausgewählt aus der Gruppe bestehend aus Alkylsulfat, Fettalkoholsulfat, Fettalkoholethersulfat und Alkylbenzolsulfonat oder deren Mischungen,
b) 0 - 20 Gew% nichtionischem Tensid, ausgewählt aus der Gruppe der Anlagerungsprodukte von 3 - 80 Mol Ethylenoxid an langkettige aliphatische Alkohole und Fettsäurealkohole mit 8 - 20 C-Atomen,
c) 0 -5 Gew % Fettsäure-amidopropyl-Betain mit einem C₅ - C₂₁-Fettsäureanteil,
d) 0 - 5 Gew% zumindest eines Lactobionofettsäureamids
e) 40 - 75 Gew% anorganischem Salz, ausgewählt aus der Gruppe bestehend aus den Alkali- und/oder Erdalkalisalzen der Schwefelsäure, Phosphorsäuren, Stickstoffsäuren, Kohlensäure oder Salzsäure oder deren Mischungen,
f) 2 - 7 Gew% Parfum
g) 0,5 - 5 Gew% Farbe in Form zumindest eines Pigmentes und/oder Farbstoffes,
h) 0 - 30 Gew% Desinfektionsmittel
i) 0 - 25 Gew% Bleichmittel,
j) 0 - 7 Gew% Aktivator,
k) 0 - 5 Gew% Komplexierungsmittel
l) 0 - 20 Gew% Abspülregulator
m) 0 - 40 Gew% Kalk- und Urinstein lösender Säure,
n) 0 - 20 Gew% Extrusionshilfsmittel,
o) 0 - 5 Gew% weiteren Zusätzen,
wobei der Gesamtanteil der Komponenten c) und d) 0,5 - 10 Gew% beträgt.

11. Mittel nach einem der Ansprüche 1 bis 10, **dadurch gekennzeichnet, dass** es zwischen 1 und 4 Gew.%, insbesondere zwischen 1 und 2,5 Gew.% wenigstens eines Lactobionofettsäureamids und / oder zwischen 0,1 und 4 Gew.%, bevorzugt zwischen 0,3 und 3 Gew.% und besonders bevorzugt zwischen 0,5 und 2,5 Gew% Betain, jeweils bezogen auf die Reinsubstanz, umfasst.

12. Verfahren zur Herstellung eines stückförmigen Mittels für den Toilettenbereich nach einem der Ansprüche 1 - 11, bei dem eine Masse entsprechender Zusammensetzung mittels eines Extruders zu einem Strang geformt und dieser in Blöcke geeigneter Größe geschnitten wird.

13. Verfahren nach Anspruch 12, **dadurch gekennzeichnet, dass** das Betain als konzentrierte wässrige Lösung mit einem Wassergehalt von weniger als 60 %, vorzugsweise weniger als 55 %, eingesetzt wird.

## Claims

1. Composition in piece form for the toilet sector, which composition has 10 to 60% by weight of anionic surfactants, 20-80% by weight of inorganic salts, fragrance and at least one fatty acyl lactobionamide and/or a betaine, the betaine, in addition to the carboxyl group and the quaternary nitrogen atom, having on at least one radical at least one heteroatom and the composition being extruded.

2. Composition according to Claim 1, **characterized in that** it is a cleansing composition in the form of a cleansing block.

3. Composition according to Claim 1, **characterized in that** it is a perfuming composition in the form of a perfume dispenser.

4. Composition according to one of Claims 1 to 3, **characterized in that** the betaine comprises an alkyl-bridged amide function.

5. Composition according to one of Claims 1 to 4, **characterized in that** the betaine is a fatty acyl amidopropylbetaine, the fatty acid having an alkyl or alkenyl radical having 5-21 carbon atoms.

6. Composition according to Claim 5, **characterized in that** the fatty acid is coconut fatty acid.

7. Composition according to Claim 1, **characterized in that** the fatty acyl lactobionamide is cocyl lactobionamide, oleyl lactobionamide, tallow lactobionamide or a mixture thereof.

8. Composition according to one of Claims 1 to 7 consisting of
a) 10-60% by weight of anionic surfactant,
b) 0-20% by weight of non-ionic surfactant,
c) 0-10% by weight of amphoteric surfactant,
d) 0-5% by weight of at least one fatty acyl lactobionamide,
e) 20-80% by weight of inorganic salt,
f) up to 10% by weight of fragrance,
g) 0-5% by weight of colour,
h) 0-30% by weight of disinfectant,
i) 0-25% by weight of bleaching agent,
j) 0-7% by weight of activator,
k) 0-5% by weight of complexing agent,
l) 0-20% by weight of rinse regulator,
m) 0-40% by weight of acid dissolving limescale and urinary salts,
n) 0-20% by weight of extrusion aid,
o) 0-5% by weight of other additives,
the total amount of components c) and d) being 0.5-10% by weight.

9. Composition according to one of Claims 1 to 8, consisting of
a) 10-50% by weight of anionic surfactant, selected from the group consisting of alkyl sulphate, fatty alcohol sulphate, fatty alcohol ether sulphate and alkylbenzene sulfonate or mixtures thereof,
b) 0-20% by weight of non-ionic surfactant, selected from the group consisting of the addition products of 3-80 mol of ethylene oxide to long-chain aliphatic alcohols and fatty acid alcohols having 8-20 carbon atoms,
c) 0-5% by weight of fatty acid amidopropylbetaine having C₅-C₂₁ fatty acid moiety,
d) 0-5% by weight of at least one fatty acyl lactobionamide,
e) 40-75% by weight of inorganic salt, selected from the group consisting of the alkali metal salts and/or alkaline earth metal salts of sulphuric acid, phosphoric acids, nitrogen acids, carbonic acid or hydrochloric acid or mixtures thereof,
f) 2-7% by weight of fragrance,
g) 0-5% by weight of colour,
h) 0-30% by weight of disinfectant,
i) 0-25% by weight of bleaching agent,
j) 0-7% by weight of activator,
k) 0-5% by weight of complexing agent,
l) 0-20% by weight of rinse regulator,
m) 0-40% by weight of acid dissolving limescale and urinary salts,
n) 0-20% by weight of extrusion aid,
o) 0-5% by weight of other additives,
the total amount of components c) and d) being 0.5-10% by weight.

10. Composition according to one of Claims 3 to 9 consisting of
a) 10-50% by weight of anionic surfactant, selected from the group consisting of alkyl sulphate, fatty alcohol sulphate, fatty alcohol ether sulphate and alkylbenzene-sulphonate or mixtures thereof,
b) 0-20% by weight of non-ionic surfactant, selected from the group consisting of the addition product of 3-80 mol of ethylene oxide to long-chain aliphatic alcohols and fatty acid alcohols having 8-20 carbon atoms,
c) 0-5% by weight of fatty acyl amidopropyl-betaine having C₅-C₂₁ fatty acid moiety,
d) 0-5% by weight of at least one fatty acyl lactobionamide,
e) 40-75% by weight of inorganic salt, selected from the group consisting of the alkali metal salts and/or alkaline earth metal salts of sulphuric acid, phosphoric acids, nitrogen acids, carbonic acid or hydrochloric acid or mixtures thereof,
f) 2-7% by weight of fragrance,
g) 0,5-5% by weight of colour, in the form of at least one pigment and/or dye,
h) 0-30% by weight of disinfectant,
i) 0-25% by weight of bleaching agent,
j) 0-7% by weight of activator,
k) 0-5% by weight of complexing agent,
l) 0-20% by weight of rinse regulator,
m) 0-40% by weight of acid dissolving limescale and urinary salts,
n) 0-20% by weight of extrusion aid,
o) 0-5% by weight of other additives,
the total amount of components c) and d) being 0.5-10% by weight.

11. Composition according to one of claims 1 to 10, **characterized in that** it comprises between 1 and 4% by weight, in particular between 1 and 2.5% by weight, of at least one fatty acyl lactobionamide and/or between 0.1 and 4% by weight, preferably between 0.3 and 3% by weight, and particularly preferably between 0.5 and 2.5% by weight, betaine, in each case based on the pure substance.

12. Process for preparing a composition in piece form for the toilet sector according to one of Claims 1 to 11, in which a composition with corresponding mass is shaped to form a rope using an extruder, and this rope is cut into blocks of suitable size.

13. Process according to Claim 12, **characterized in that** the betaine is used as concentrated aqueous solution having a water content of less than 60%, preferably less than 55%.

## Revendications

1. Agent sous forme de morceau pour le domaine de la toilette, lequel agent contient de 10 à 60 % en poids d'agents tensioactifs anioniques, de 20 à 80 % en poids de sels inorganiques, du parfum et au moins un amide d'acide gras lactobionique et/ou une bétaïne, la bétaïne présentant, en sus du groupement carboxy et de l'atome d'azote quaternaire sur au moins un radical, au moins un hétéro-atome et l'agent étant extrudé.

2. Agent selon la revendication 1, **caractérisé en ce qu'**il s'agit d'un agent de nettoyage sous la forme d'un bloc de nettoyage.

3. Agent selon la revendication 1, **caractérisé en ce qu'**il s'agit d'un agent parfumant sous la forme d'un distributeur de parfum.

4. Agent selon l'une quelconque des revendications 1 à 3, **caractérisé en ce que** la bétaïne inclut une fonction amide à pont alkyle.

5. Agent selon l'une quelconque des revendications 1 à 4, **caractérisé en ce que** la bétaïne est une bétaïne amidopropyle d'acide, gras, l'acide gras présentant un radical alkyle ou alcényle ayant 5 - 21 atomes de carbone.

6. Agent selon la revendication 5, **caractérisé en ce que** l'acide gras est l'acide gras d'huile de noix de coco.

7. Agent selon la revendication 1, **caractérisé en ce que** l'amide d'acide gras lactobionique est l'amide de lactobionococyle, l'amide de lactobionooléyle, l'amide de lactobionotalge ou un mélange de ces derniers.

8. Agent selon l'une quelconque des revendications 1 à 7, se composant
a) de 10 - 60 % en poids d'agent tensioactif anionique,
b) de 0 - 20 % en poids d'agent tensioactif non ionique,
c) de 0 - 10 % en poids d'agent tensioactif amphotère,
d) de 0 - 5 % en poids d'au moins un amide d'acide gras lactobionique,
e) de 20 - 80 % en poids de sel inorganique,
f) jusqu'à 10 % en poids de parfum,
g) de 0 - 5 % en poids de colorant,
h) de 0 - 30 % en poids d'agent désinfectant,
i) de 0 - 25 % en poids d'agent de blanchiment,
j) de 0 - 7 % en poids d'agent activateur,
k) de 0 - 5 % en poids d'agent complexant,
l) de 0 - 20 % en poids d'agent de régulation du rinçage,
m) de 0 - 40 % en poids d'acide dissolvant les dépôts de calcaire et d'urine,
n) de 0 - 20 % en poids d'agent adjuvant d'extrusion,
o) de 0 - 5 % en poids d'additifs supplémentaires,
la proportion globale des composants c) et d) étant de 0,5 à 10 % en poids.

9. Agent selon l'une quelconque des revendications 1 à 8, se composant
a) de 10 - 50 % en poids d'agent tensioactif anionique, sélectionné parmi le groupe constitué de sulfate alkyle, de sulfate d'alcool gras, d'éthersulfate d'alcool gras et d'alkylbenzènesulfonate ou de leurs mélanges,
b) de 0 - 20 % en poids d'agent tensioactif non ionique, sélectionné parmi le groupe des produits d'addition de 3 - 80 moles d'oxyde d'éthylène sur des alcools aliphatiques à longue chaîne et des alcools d'acide gras, ayant 8 - 20 atomes de C,
c) de 0 - 5 % en poids de bétaïne amidopropyle d'acide gras ayant une proportion d'acide gras en C₅-C₂₁,
d) de 0 - 5 % en poids d'au moins un amide d'acide gras lactobionique,
e) de 40 - 75 % en poids de sel inorganique, sélectionné parmi le groupe constitué de sels alcalins et/ou alcalino-terreux de l'acide sulfurique, des acides du phosphore, des acides de l'azote, de l'acide carbonique ou de l'acide chlorhydrique ou de leurs mélanges,
f) de 2 - 7 % en poids de parfum,
g) de 0 - 5 % en poids de colorant,
h) de 0 - 30 % en poids d'agent désinfectant,
i) de 0 - 25 % en poids d'agent de blanchiment,
j) de 0 - 7 % en poids d'agent activateur,
k) de 0 - 5 % en poids d'agent complexant,
l) de 0 - 20 % en poids d'agent de régulation du rinçage,
m) de 0 - 40 % en poids d'acide dissolvant les dépôts de calcaire et d'urine,
n) de 0 - 20 % en poids d'agent adjuvant d'extrusion,
o) de 0 - 5 % en poids d'additifs supplémentaires,
la proportion globale des composants c) et d) étant de 0,5 à 10 % en poids.

10. Agent selon l'une quelconque des revendications 3 à 9, se composant
a) de 10 - 50 % en poids d'agent tensioactif anionique, sélectionné parmi le groupe constitué de sulfate alkyle, de sulfate d'alcool gras, d'éthersulfate d'alcool gras et d' alkyle benzènesulfonate ou de leurs mélanges,
b) de 0 - 20 % en poids d'agent tensioactif non ionique, sélectionné parmi le groupe des produits d'addition de 3 - 80 moles d'oxyde d'éthylène sur des alcools aliphatiques à longue chaîne et des alcools d'acide gras, ayant 8 - 20 atomes de C,
c) de 0 - 5 % en poids de bétaïne amidopropyle d'acide gras ayant une proportion d'acide gras en C₅-C₂₁,
d) de 0 - 5 % en poids d'au moins un amide d'acide gras lactobionique,
e) de 40 - 75 % en poids de sel inorganique, sélectionné parmi le groupe constitué de sels alcalins et/ou alcalino-terreux de l'acide sulfurique, des acides du phosphore, des acides de l'azote, de l'acide carbonique ou de l'acide chlorhydrique ou de leurs mélanges,
f) de 2 - 7 % en poids de parfum,
g) de 0,5 - 5 % en poids de colorant sous la forme d'au moins un pigment et/ou un colorant,
h) de 0 - 30 % en poids d'agent désinfectant,
i) de 0 - 25 % en poids d'agent de blanchiment,
j) de 0 - 7 % en poids d'agent activateur,
k) de 0 - 5 % en poids d'agent complexant,
l) de 0 - 20 % en poids d'agent de régulation du rinçage,
m) de 0 - 40 % en poids d'acide dissolvant les dépôts de calcaire et d'urine,
n) de 0 - 20 % en poids d'agent adjuvant d'extrusion,
o) de 0 - 5 % en poids d'additifs supplémentaires,
la proportion globale des composants c) et d) étant de 0,5 à 10 % en poids.

11. Agent selon l'une quelconque des revendications 1 à 10, **caractérisé en ce qu'**il inclut entre 1 et 4 % en poids, en particulier entre 1 et 2,5 % en poids d'au moins un amide d'acide gras lactobionique et/ou entre 0,1 et 4 % en poids, de préférence, entre 0,3 et 3 % en poids et, en particulier, de préférence, entre 0,5 et 2,5 % en poids de bétaïne, à chaque fois, par rapport à la substance pure.

12. Procédé en vue de la fabrication d'un agent en forme de morceau pour le domaine de la toilette selon l'une quelconque des revendications 1 à 11, lors duquel une masse de composition correspondante est formée à l'aide d'une extrudeuse pour donner un boudin et lors duquel celui-ci est coupé en blocs d'une taille appropriée.

13. Procédé selon la revendication 12, **caractérisé en ce que** la bétaïne est utilisée en tant que solution aqueuse concentrée ayant une teneur en eau de moins de 60 %, de préférence, de moins de 55 %.
